# EUROPEAN PATENT SPECIFICATION

(11) **EP 4 257 068 B1**
(45) Date of publication and mention of the grant of the patent: **22.07.2026**
(21) Application number: 23165832.9
(22) Date of filing: 31.03.2023
(51) Int. Cl.: A61B 18/14, A61B 34/20

(54) **CATHETER WITH EXTERNAL MAGNETIC COILS**
KATHETER MIT ÄUSSEREN MAGNETSPULEN
CATHÉTER AVEC BOBINES MAGNÉTIQUES EXTERNES

(30) Priority: 05.04.2022 US 202263327460 P; 21.03.2023 US 202318187497
(43) Date of publication of application: 11.10.2023
(73) Proprietor: BIOSENSE WEBSTER (ISRAEL) LTD., 2066717 Yokneam (IL)
(72) Inventor: VAN NIEKERK, Pieter E., Irvine, 92618 (US); EBRAHIMI, Babak, Irvine, 92618 (US); OVIEDO BUITRAGO, Andrea L., Irvine, 92618 (US); HENRIQUEZ, Jamie, Irvine, 92618 (US)
(74) Representative: Carpmaels & Ransford LLP

(56) References cited:
- EP-B1- 0 910 278
- WO-A1-2020/104888
- US-A1- 2018 228 392
- US-A1- 2019 038 228
- US-A1- 2021 077 183

## Description

### BACKGROUND

Cardiac arrhythmias, such as atrial fibrillation, occur when regions of cardiac tissue abnormally conduct electric signals. Procedures for treating arrhythmia include surgically disrupting the conducting pathway for such signals. By selectively ablating cardiac tissue by application of energy (e.g., radiofrequency (RF) energy), it may be possible to cease or modify the propagation of unwanted electrical signals from one portion of the heart to another. The ablation process may provide a barrier to unwanted electrical pathways by creating electrically insulative lesions or scar tissue that effectively block communication of aberrant electrical signals across the tissue.

In some procedures, a catheter with one or more RF electrodes may be used to provide ablation within the cardiovascular system. The catheter may be inserted into a major vein or artery (e.g., the femoral artery) and then advanced to position the electrodes within the heart or in a cardiovascular structure adjacent to the heart (e.g., the pulmonary vein). The one or more electrodes may be placed in contact with cardiac tissue or other vascular tissue and then activated with RF energy to thereby ablate the contacted tissue. In some cases, the electrodes may be bipolar. In some other cases, a monopolar electrode may be used in conjunction with a ground pad or other reference electrode that is in contact with the patient that is in contact with the patient. Irrigation may be used to draw heat from ablating components of an ablation catheter; and to prevent the formation of blood clots near the ablation site.

Examples of ablation catheters are described in U.S. Pat. No. 10,743,932, entitled "Integrated Ablation System using Catheter with Multiple Irrigation Lumens," issued August 18, 2020; U.S. Pat. No. 10,660,700, entitled "Irrigated Balloon Catheter with Flexible Circuit Electrode Assembly," issued May 26, 2020; U.S. Pub. No. 2018/0071017, entitled "Ablation Catheter with a Flexible Printed Circuit Board," published March 15, 2018; U.S. Pat. No. 10,702,177, entitled "Catheter with Bipole Electrode Spacer and Related Methods," issued July 7, 2020; U.S. Pat. No. 10,130,422, entitled "Catheter with Soft Distal Tip for Mapping and Ablating Tubular Region," issued November 20, 2018; U.S. Pat. No. 8,956,353, entitled "Electrode Irrigation Using Micro-Jets," issued February 17, 2015; and U.S. Pat. No. 9,801,585, entitled "Electrocardiogram Noise Reduction," issued October 31, 2017

Some catheter ablation procedures may be performed after using electrophysiology (EP) mapping to identify tissue regions that should be targeted for ablation. Such EP mapping may include the use of sensing electrodes on a catheter (e.g., the same catheter that is used to perform the ablation or a dedicated mapping catheter). Such sensing electrodes may monitor electrical signals emanating from conductive endocardial tissues to pinpoint the location of aberrant conductive tissue sites that are responsible for the arrhythmia. Examples of an EP mapping system are described in U.S. Pat. No. 5,738,096, entitled "Cardiac Electromechanics," issued April 14, 1998. Examples of EP mapping catheters are described in U.S. Pat. No. 9,907,480, entitled "Catheter Spine Assembly with Closely-Spaced Bipole Microelectrodes," issued March 6, 2018; U.S. Pat. No. 10,130,422, entitled "Catheter with Soft Distal Tip for Mapping and Ablating Tubular Region," issued November 20, 2018and U.S. Pat. No. 10,702,177, entitled "Catheter with Bipole Electrode Spacer and Related Methods," issued July 7, 2020.

In addition to using EP mapping, some catheter ablation procedures may be performed using an image guided surgery (IGS) system. The IGS system may enable the physician to visually track the location of the catheter within the patient, in relation to images of anatomical structures within the patient, in real time. Some systems may provide a combination of EP mapping and IGS functionalities, including the CARTO 3^{®} system by Biosense Webster, Inc. of Irvine, California. Examples of catheters that are configured for use with an IGS system are disclosed in U.S. Pat. No. 9,480,416, entitled "Signal Transmission Using Catheter Braid Wires," issued November 1, 2016; and various other references that are cited herein.
In US 2021/077183 A1, there is described an apparatus including a catheter and an end effector. At least a portion of the catheter is sized and configured to fit within a lumen of a cardiovascular system. The end effector is positioned at a distal end of the catheter. The end effector includes a panel, a plurality of mapping electrodes positioned on a first surface of the panel, and a plurality of ablation electrodes positioned on the first surface of the panel.
In US 2019/038228 A1, there are described systems and methods for detecting force applied to a distal tip of a medical catheter. In some embodiments, a medical catheter with a deformable body near a distal tip of the catheter deforms in response to a force applied at the distal tip, and a force sensor detects various components of the deformation.
In US 2018/228392 A1, there is described a position sensor including a flexible substrate formed into a three-dimensional (3D) shape. At least first and second field-sensing coils are formed in first and second respective layers of the flexible substrate, such that in the 3D shape the first and second field-sensing coils have first and second respective axes that are not parallel to one another.
In WO 2020/104888 A1, there is described an apparatus including a catheter shaft and an end effector at a distal end of the catheter shaft. The end effector is sized to fit in an anatomical passageway within a cardiovascular system. The end effector includes a plurality of circuit boards and a distal tip member. The circuit boards are stacked longitudinally along a longitudinal axis of the end effector to define a stack of circuit boards and are configured to communicate electrical signals along a full length of the stack of circuit boards.
In EP 0910278 A1, there is described a catheter with a lumen wherein the lumen is obstructed by a portion of the catheter. The catheter includes a position detector at the tip of the catheter.

While several catheter systems and methods have been made and used, it is believed that no one prior to the inventors has made or used the invention described in the appended claims.

### BRIEF DESCRIPTION OF THE DRAWINGS

The drawings and detailed description that follow are intended to be merely illustrative and are not intended to limit the scope of the invention as contemplated by the inventors.
FIG. 1 depicts a schematic view of a medical procedure in which a catheter of a catheter assembly is inserted in a patient;
FIG. 2A depicts a perspective view of the catheter assembly of FIG. 1, with an end effector of the catheter in a proximal position relative to an outer sheath of the catheter;
FIG. 2B depicts a perspective view of the catheter assembly of FIG. 1, with the end effector in a distal position relative to the outer sheath;
FIG. 3 depicts a plan view of the end effector of FIG. 2B;
FIG. 4 depicts a plan view of an example of a sensor assembly that may be incorporated at a distal end of the catheter assembly of FIG. 1 and the proximal end of the end effector of FIG. 2B;
FIG. 5 depicts a plan view of another example of a sensor assembly that may be incorporated at a distal end of the catheter assembly of FIG. 1 and the proximal end of the end effector of FIG. 2B; and
FIG. 6 depicts a plan view of another example of a sensor assembly that may be incorporated at a distal end of the catheter assembly of FIG. 1 and the proximal end of the end effector of FIG. 2B.

### DETAILED DESCRIPTION OF EMBODIMENTS

The invention provides an apparatus according to claim 1. Further embodiments of the invention are described in the dependent claims. The following description of certain examples of the invention should not be used to limit the scope of the present invention. The drawings, which are not necessarily to scale, depict selected embodiments and are not intended to limit the scope of the invention. The detailed description illustrates by way of example, not by way of limitation, the principles of the invention. Other examples, features, aspects, embodiments, and advantages of the invention will become apparent to those skilled in the art from the following description, which is by way of illustration, one of the best modes contemplated for carrying out the invention. As will be realized, the invention is capable of other different or equivalent aspects, all without departing from the invention. Accordingly, the drawings and descriptions should be regarded as illustrative in nature and not restrictive.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

As used herein, the terms "generally," "substantially," "about," or "approximately" for any numerical values or ranges indicate a suitable dimensional tolerance that allows the part or collection of components to function for its intended purpose as described herein. In addition, as used herein, the terms "patient," "host," "user," and "subject" refer to any human or animal subject and are not intended to limit the systems or methods to human use, although use of the subject invention in a human patient represents a preferred embodiment.

### I. Overview of Example of a Catheter System

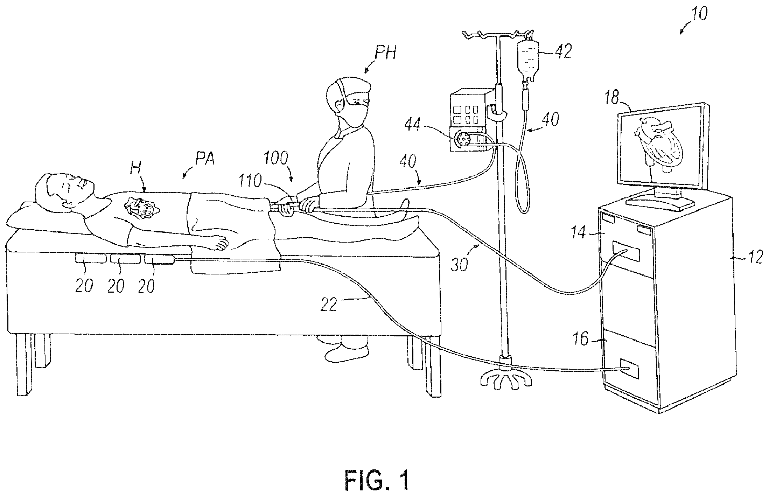

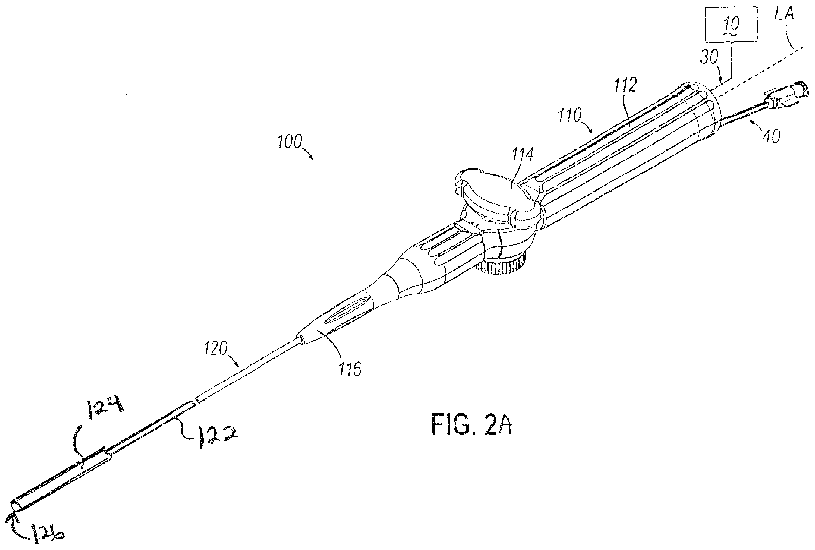

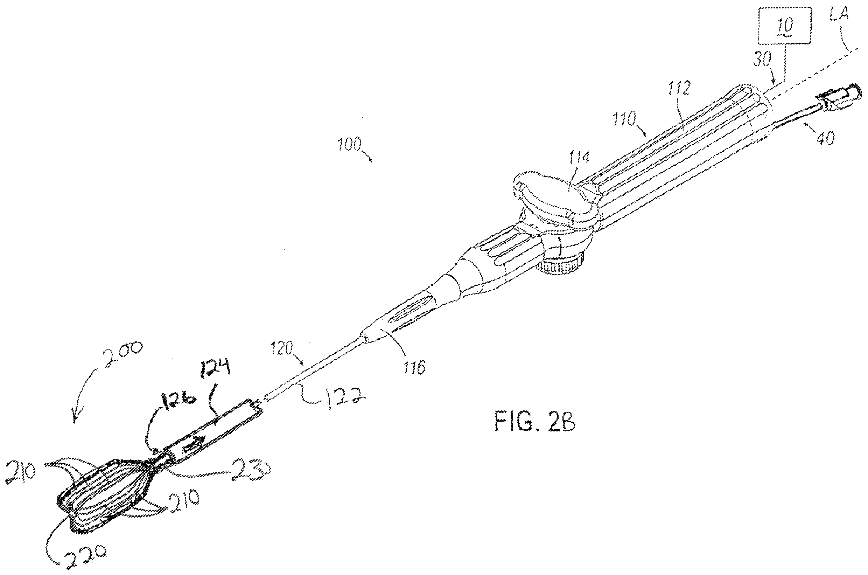

FIG. 1 shows an exemplary medical procedure and associated components of a cardiac catheter system that may be used to provide EP mapping or cardiac ablation as referred to above. In particular, FIG. 1 shows a physician (PH) grasping a handle assembly (110) of a catheter assembly (100), with an end effector (200) (shown in FIG. 2B but not shown in FIG. 1) of a flexible catheter (120) (shown in FIGS. 2A-2B but not shown in FIG. 1) of catheter assembly (100) disposed in a patient (PA) to map potentials in tissue or ablate tissue in or near the heart (H) of the patient (PA). As shown in FIGS. 2A-2B, catheter assembly (100) includes handle assembly (110), catheter (120) extending distally from handle assembly (110), end effector (200) located at a distal end of catheter (120), and a deflection drive actuator (114) associated with handle assembly (110). Deflection drive actuator (114) is rotatable relative to a casing (112) of handle assembly (110) to thereby deflect end effector (140) and a distal portion of catheter (120) away from a central longitudinal axis (LA) defined by a proximal portion of catheter (120). Various suitable components that may be coupled with deflection drive actuator (114) and catheter (120) to provide such functionality will be apparent to those skilled in the art in view of the teachings herein.

Catheter (120) includes an elongate flexible shaft (122) and an outer sheath (124). Shaft (122) is coaxially and slidably disposed within outer sheath (124). End effector (200) is positioned at the distal end of shaft (122). The proximal end of catheter (120) extends distally from a nozzle member (116) of handle assembly (110). In some versions, outer sheath (124) is an integral component of catheter (120). In some other versions, sheath (124) is a component of another instrument; and catheter (120) is inserted into sheath (124). In either scenario, shaft (122) and an outer sheath (124) may transition between two arrangements, including a first arrangement as shown in FIG. 2A where outer sheath (124) is distally positioned in relation to shaft (122), such that end effector (200) is contained within outer sheath (124); and a second arrangement as shown in FIG. 2B where outer sheath (124) is proximally positioned in relation to shaft (122), such that end effector (200) is exposed relative to outer sheath (124). In some versions, shaft (122) translates relative to handle assembly (110) to achieve the different longitudinal positioning relative to outer sheath (124). In some other versions, outer sheath (124) translates relative to handle assembly (110) to achieve the different longitudinal positioning relative to shaft (122).

Catheter (120) and end effector (200) may be in the state shown in FIG. 2A when catheter (120) is introduced into the body of the patient (PA); and during transit from the insertion site to the targeted cardiovascular region within the patient (PA). Once catheter (120) and end effector (200) reach the targeted cardiovascular region within the patient (PA), outer sheath (124) may be retracted proximally to expose end effector (200), thereby allowing end effector (200) to achieve the expanded, deployed state shown in FIG. 2B.

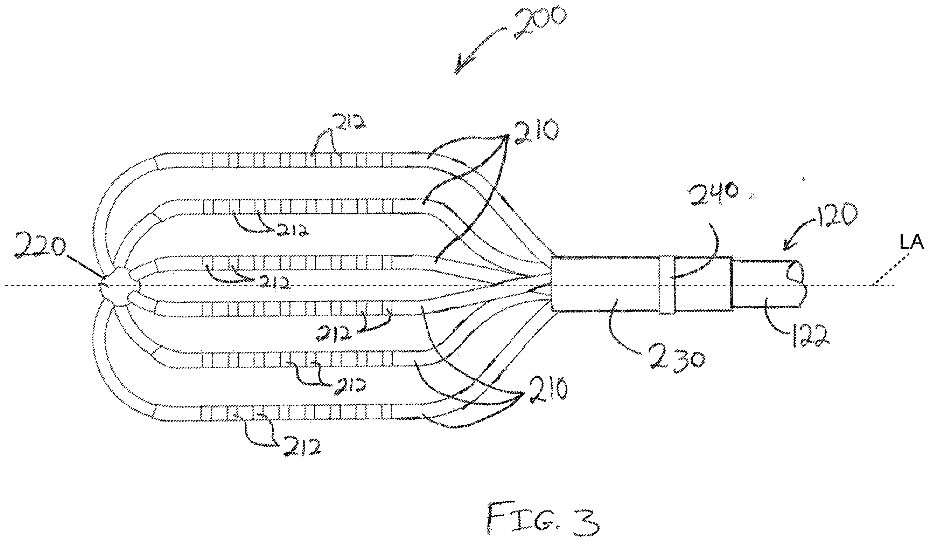

As shown in FIG. 2B and FIG. 3, end effector (200) is secured to the distal end of shaft (122) of catheter (120) via a coupling member (230). End effector (200) of the present example includes a plurality of spines (210). The proximal portion of each spine (210) diverges outwardly away from the central longitudinal axis (LA) defined by a proximal portion of catheter (120). The longitudinally intermediate portion of each spine (210) is generally parallel with the central longitudinal axis (LA). The distal portion of each spine (210) converges back toward the central longitudinal axis (LA). In particular, each spine (210) is distally joined to a distal coupling (220). In the present example, distal coupling (220) is in the form of a ball. Alternatively, distal coupling (220) may take any other suitable form. Each spine (210) also includes a plurality of electrodes (212) that are spaced apart from each other along the length of spine (210). In some versions, electrodes (212) are operable to provide EP mapping. In addition, or in the alternative, electrodes (212) may be operable to ablate tissue. Spines (210) may also include various other features, such as position sensors, temperature sensors, etc.

Spines (210) may be formed of a resilient material (e.g., nitinol, etc.) such that spines (210) may be resiliently biased to assume the configuration shown in FIG. 2B and FIG. 3. In some versions, end effector (200) defines a generally planar configuration when spines (210) are in the state shown in FIG. 2B and FIG. 3, with the plane defined by end effector (210) extending along and transversely from the longitudinal axis (LA). Spines (210) are also deformable such that spines (210) may be contained within outer sheath (124) as shown in FIG. 2A. Spines (210) may thus deform inwardly toward the longitudinal axis (LA), along the plane defined by end effector (200), to fit within sheath (124). In addition to deforming and expanding along the plane defined by end effector (200) to transition between the state shown in FIG. 2A and the state shown in FIG. 2B and FIG. 3, spines (210) may also deform along a direction that is transverse to the plane defined by end effector (200). Such deformation may occur as end effector (200) is pressed against tissue.

Coupling member (230) may take any suitable form. Some versions of coupling member (230) comprise a cylindrical plastic (e.g., polyether ether ketone (PEEK), etc.) component. The proximal ends of spines (210) may be fixedly secured to coupling member (230) via adhesive, overmolding, welding, epoxy, or in any other suitable fashion. Similarly, the distal end of flexible shaft (122) may be fixedly secured to coupling member (230) via adhesive, overmolding, welding, epoxy, or in any other suitable fashion. In some versions, the distal end of flexible shaft (122) is fixedly secured to the proximal ends of spines (210) within coupling member (230), in addition to these components being fixedly secured to coupling member (230). Coupling member (230) of the present example also includes a ring electrode (240). In some versions, ring electrode (240) is used to provide a reference signal from blood of a patient while electrodes (212) provide EP mapping signals from tissue of the patient. Alternatively, ring electrode (240) may be used for any other suitable purpose(s). In some variations, ring electrode (240) is omitted.

Catheter assembly (100) is coupled with a guidance and drive system (10) via a cable (30). Catheter assembly (100) is also coupled with a fluid source (42) via a fluid conduit (40), though this is optional. A set of field generators (20) are positioned underneath the patient (PA) and are also coupled with guidance and drive system (10) via a cable (22). Guidance and drive system (10) of the present example includes a console (12) and a display (18). Console (12) includes a first driver module (14) and a second driver module (16). First driver module (14) is coupled with catheter assembly (100) via cable (30). In some variations, first driver module (14) is operable to receive EP mapping signals obtained via electrodes (212) of end effector (200); and a reference signal from ring electrode (240). Console (12) includes a processor (not shown) that processes such EP mapping signals and thereby provides EP mapping as is known in the art. In addition, or in the alternative, first driver module (14) may be operable to provide RF power to electrodes (212) of end effector (200) to thereby ablate tissue.

In some versions, first driver module (14) is also operable to receive position indicative signals from one or more position sensors of catheter assembly (100), as will be described in greater detail below. In such versions, the processor of console (12) is also operable to process the position indicative signals from position sensors to thereby determine the position of end effector (200) or other components of catheter assembly (100) within the patient (PA). Other components and techniques that may be used to generate real-time position data associated with end effector (200) may include wireless triangulation, acoustic tracking, optical tracking, inertial tracking, and the like. By way of further example only, alternative position sensing may include impedance measurement-based position sensing. Examples of such position sensing are described in U.S. Pat. No. 7,869,865, entitled "Current-Based Position Sensing," issued January 11, 2011; and U.S. Pat. No. 8,456,182, entitled "Current Localization Tracker," issued June 4, 2013. Alternatively, any other suitable position sensing technologies and techniques may be used.

Second driver module (16) is coupled with field generators (20) via cable (22). Second driver module (16) is operable to activate field generators (20) to generate an alternating magnetic field around the heart (H) of the patient (PA). For instance, field generators (20) may include coils that generate alternating magnetic fields in a predetermined working volume that contains the heart (H).

Display (18) is coupled with the processor of console (12) and is operable to render images of patient anatomy. Such images may be based on a set of preoperatively or intraoperatively obtained images (e.g., a CT or MRI scan, 3-D map, etc.). The views of patient anatomy provided through display (18) may also change dynamically based on signals from position sensors of catheter assembly (100). For instance, as end effector (200) of catheter (120) moves within the patient (PA), the corresponding position data from position sensors may cause the processor of console (12) to update the patient anatomy views in display (18) in real time to depict the regions of patient anatomy around end effector (200) as end effector (200) moves within the patient (PA). Moreover, the processor of console (12) may drive display (18) to show locations of aberrant conductive tissue sites, as detected via EP mapping with end effector (200). By way of example only, the processor of console (12) may drive display (18) to superimpose the locations of aberrant conductive tissue sites on the images of the patient's anatomy, such as by superimposing an illuminated dot, a crosshair, or some other form of visual indication of aberrant conductive tissue sites.

The processor of console (12) may also drive display (18) to superimpose the current location of end effector (200) on the images of the patient's anatomy, such as by superimposing an illuminated dot, a crosshair, a graphical representation of end effector (200), or some other form of visual indication. Such a superimposed visual indication may also move within the images of the patient anatomy on display (18) in real time as the physician moves end effector (200) within the patient (PA), thereby providing real-time visual feedback to the operator about the position of end effector (200) within the patient (PA) as end effector (200) moves within the patient (PA). The images provided through display (18) may thus effectively provide a video tracking the position of end effector (200) within a patient (PA), without necessarily having any optical instrumentation (i.e., cameras) viewing end effector (200). In the same view, display (18) may simultaneously visually indicate the locations of aberrant conductive tissue sites detected through the EP mapping. The physician (PH) may thus view display (18) to observe the real time positioning of end effector (200) in relation to the mapped aberrant conductive tissue sites and in relation to images of the adjacent anatomical structures in the patient (PA).

Fluid source (42) of the present example includes a bag containing saline or some other suitable irrigation fluid. Conduit (40) includes a flexible tube that is further coupled with a pump (44), which is operable to selectively drive fluid from fluid source (42) to catheter assembly (100). In some variations, conduit (40), fluid source (42), and pump (44) are omitted entirely. In versions where these components are included, end effector (200) may be configured to communicate irrigation fluid from fluid source (42) to the target site in the patient. Such irrigation may be provided in accordance with the teachings of any of the various patent references cited herein; or in any other suitable fashion as will be apparent to those skilled in the art in view of the teachings herein.

### II. Examples of Position Sensor Assemblies

As noted above, one or more features of catheter assembly (100) may include one or more position sensors. The processor of console (12) may process the position indicative signals from such position sensors to thereby determine the real-time position of end effector (200) or other components of catheter assembly (100) within the patient (PA). In some scenarios, it may be desirable to incorporate such position sensors within end effector (200), to thereby enable determination of the real-time position of one or more portions of end effector (200) within the patient (PA).

In addition to including one or more position sensors within end effector (200), or in lieu of including one or more position sensors within end effector (200), it may be desirable to include one or more position sensors at or near the distal end of flexible shaft (122). For instance, in versions where size and space constraints do not permit incorporation of position sensors in end effector (200), a position sensor at or near the distal end of flexible shaft (122) may at least effectively provide an indication of the real-time position of the proximal end of end effector (200). Even in versions where one or more position sensors is/are included in end effector (200), it still may be desirable to include one or more position sensors at or near the distal end of flexible shaft (122) to thereby enable determination of the real-time position of the distal end of flexible shaft (122) within the patient (PA). Such position information may be particularly useful in instances where end effector (200) is deflected away from the longitudinal axis (LA), where real-time position data from position sensors in end effector (200) might not adequately provide determination of the real-time position of the distal end of flexible shaft (122).

With coupling member (230) being located at the proximal end of end effector (200) and at the distal end of flexible shaft (122), coupling member (230) may provide a suitable location for one or more position sensors that may enable determination of the real-time position of both the proximal end of end effector (200) and the distal end of flexible shaft (122). However, the interior region of coupling member (230) may not have enough space to adequately accommodate one or more position sensors. Even if the interior region of coupling member (230) were large enough to accommodate one or more position sensors, it may still be desirable to instead position such position sensor(s) on the exterior region of coupling member (230) to leave room within the interior region of coupling member (230) for other components (e.g., an irrigation conduit, structural reinforcement features, etc.). It may therefore be desirable to incorporate one or more position sensors about the exterior region of coupling member (230). Even if the interior region of coupling member (230) were large enough to accommodate one or more position sensors, it may still be desirable to instead position such position sensor(s) on the exterior region of coupling member (230) to leave room within the interior region of coupling member (230) for other components (e.g., an irrigation conduit, structural reinforcement features, etc.). Positioning one or more position sensor(s) on the exterior region of coupling member (230) may be done in such a way that does not ultimately result in unacceptable enlargement of the outer diameter of coupling member (230).

In addition to avoiding constraints that might otherwise prevent or render difficult the positioning of a position sensor within an interior region of coupling member (230), positioning the position sensor on the exterior region of coupling member (230) may provide a larger surface area for the position sensor, which may in turn provide a greater position-sensing sensitivity to the position sensor.

The following describes various examples of how one or more position sensors may be incorporated about the exterior region of a coupling member like coupling member (230). While the following examples are provided in the context of end effector (200), the following teachings may be readily applied to catheter assemblies (100) that have any other suitable kind of end effector. For instance, the following teachings may be readily applied to catheter assemblies (100) that have end effectors that are constructed in accordance with the teachings of any of the various patent references cited herein; or any other suitable kinds of end effectors.

### 1. Example of Single-Axis Position Sensor Assembly

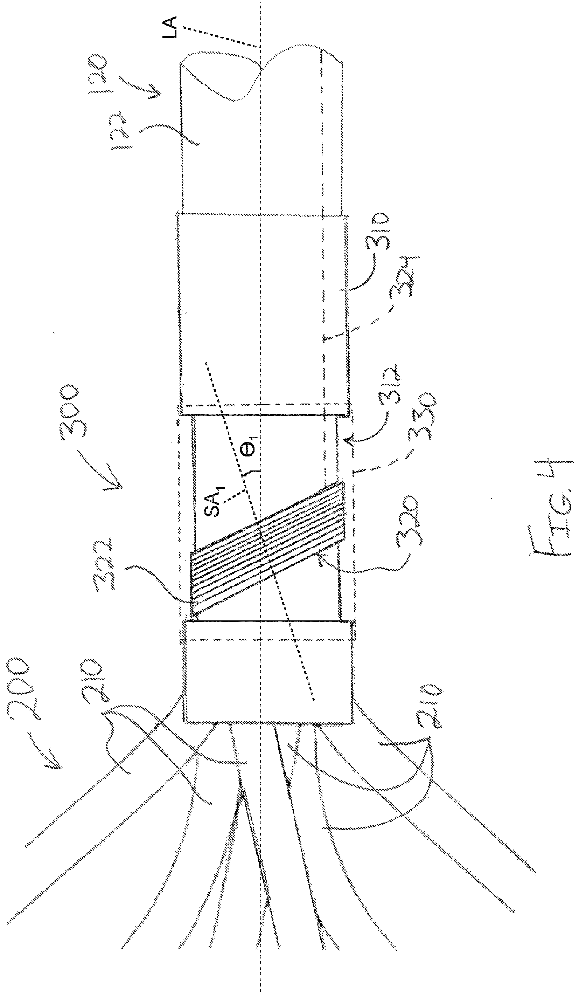

FIG. 4 shows an example of a sensor assembly (300) that may be incorporated into a variation of catheter assembly (100). Sensor assembly (300) of this example includes a coupling member (310) and a coil (320). Coupling member (310) may be constructed and operable like coupling member (230) described above. Coupling member (310) is fixedly secured with the distal end of flexible catheter (122) and the proximal end of end effector (200). In some versions, coupling member (310) is in the form of a rigid plastic (e.g., polyether ether ketone) body. In versions where coupling member (310) is in the form of a rigid body, and catheter shaft (122) includes a steerable section that may be deflected using deflection drive actuator (114), it may be desirable to minimize the length of coupling member (310) in order to bring end effector (200) as close as possible to the point of deflection at this steerable section. Unlike coupling member (230), coupling member (310) of this example includes an exterior recess (312), along which the outer diameter of coupling member (310) is less than the outer diameter of the rest of coupling member (310). In some versions, the reduction in diameter at exterior recess (312) (i.e., the depth of exterior recess (312)) is substantially equal to, or otherwise corresponds with, a thickness of coil (320).

Coil (320) of the present example comprises a wire (322) that is wrapped around the exterior of coupling member (310), within exterior recess (312). In some other versions, coil (320) comprises a flex circuit that includes a combination of a flexible substrate wrapped around the exterior of coupling member (310) and one or more conductive traces on the flexible substrate, where those conductive traces form a coil shape. Coil (320) is configured to generate electrical signals in response to the presence of alternating magnetic fields that are generated by field generators (20). Such electrical signals from coil (320) may indicate the real-time position of coil (320) within the patient (PA).

The electrical signals generated in coil (320) are communicated along an electrical conduit (324) and cable (30) to reach the processor of console (12). Electrical conduit (324) extends along the length of catheter (120) and may electrically couple with cable (30) in any suitable fashion. In some versions, electrical conduit (324) comprises one or more wires. In addition, or in the alternative, electrical conduit (324) may include one or more traces formed on a substrate of a flex circuit. Alternatively, electrical conduit (324) may take any other suitable form. In some versions, electrical conduit (324) extends along the interior of flexible shaft (122). In addition, or in the alternative, a portion or all of electrical conduit (324) may extend along an exterior of flexible shaft (122).

Coil (320) of this example is oriented such that the windings of coil (320) extend around a first sensor axis (SA₁). First sensor axis (SA₁) is oriented obliquely relative to the longitudinal axis (LA), such that first sensor axis (SA₁) and the longitudinal axis (LA) together define an oblique first angle (Θ₁). By way of example only, first angle (Θ₁) may range from approximately 5 degrees to approximately 45 degrees. Alternatively, any other suitable first angle (Θ₁) may be provided. Because first angle (Θ₁) is oblique in the present example, coil (320) is configured to generate electrical signals that indicate a "roll" angular position of catheter (120) and end effector (200) about the longitudinal axis (LA). In other words, if first sensor axis (SA₁) were coaxial with the longitudinal axis (LA), coil (320) the electrical signal generated by coil (320) may not indicate the "roll" angular position of catheter (120) and end effector (200) about the longitudinal axis (LA).

Sensor assembly (300) of the present example further includes an outer layer (330) that is positioned about coupling member (310) and coil (320). In some versions, outer layer (330) extends along the full length of coupling member (310). In the present example, outer layer (330) extends along a length that is greater than the length of exterior recess (312) yet less than the full length of coupling member (310). Outer layer (330) is configured and positioned to cover coil (320) and exterior recess (312) without interfering with the functionality of coil (320). Outer layer (330) may prevent edges of exterior recess (312) from snagging or outer layer (330) may serve any other suitable purpose(s). In some versions, outer layer (330) includes a heat-shrink wrap. Alternatively, outer layer (330) may take any other suitable form.

### 2. Example of Two-Axis Position Sensor Assembly

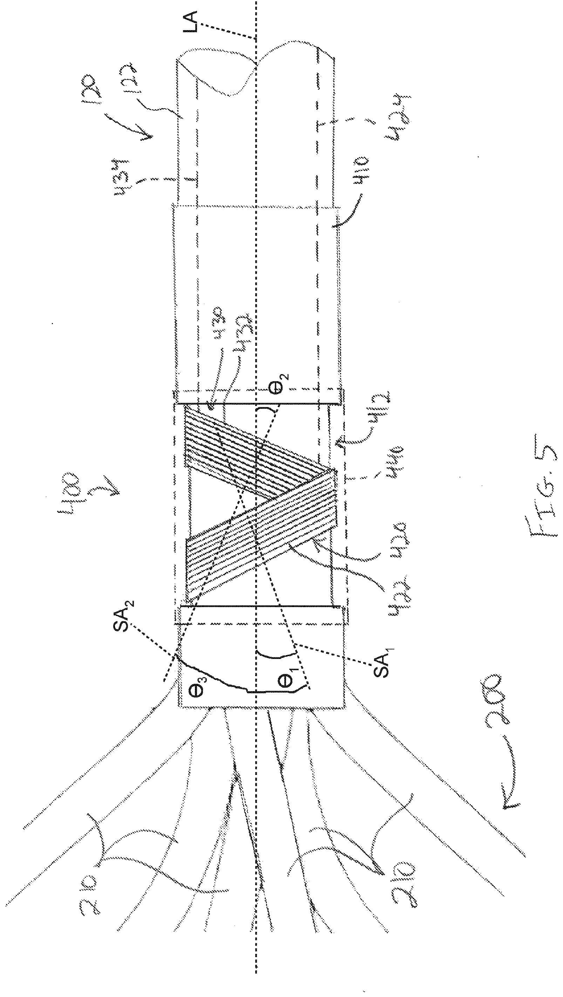

FIG. 5 shows an example of another sensor assembly (400) that may be incorporated into a variation of catheter assembly (100). Sensor assembly (400) of this example includes a coupling member (410), a first coil (420), and a second coil (430). Coupling member (410) may be constructed and operable like coupling member (230) described above. Coupling member (410) is fixedly secured with the distal end of flexible catheter (122) and the proximal end of end effector (200). In some versions, coupling member (410) is in the form of a rigid plastic (e.g., polyether ether ketone) body. In versions where coupling member (410) is in the form of a rigid body, and catheter shaft (122) includes a steerable section that may be deflected using deflection drive actuator (114), it may be desirable to minimize the length of coupling member (310) in order to bring end effector (200) as close as possible to the point of deflection at this steerable section. Unlike coupling member (230), coupling member (410) of this example includes an exterior recess (412), along which the outer diameter of coupling member (410) is less than the outer diameter of the rest of coupling member (410). In some versions, the reduction in diameter at exterior recess (412) (i.e., the depth of exterior recess (412)) is substantially equal to, or otherwise corresponds with, a thickness of coils (420, 430).

First coil (420) of the present example comprises a wire (422) that is wrapped around the exterior of coupling member (410), within exterior recess (412). In some other versions, first coil (420) comprises a flex circuit that includes a combination of a flexible substrate wrapped around the exterior of coupling member (410) and one or more conductive traces on the flexible substrate, where those conductive traces form a coil shape. First coil (420) is configured to generate electrical signals in response to the presence of alternating magnetic fields that are generated by field generators (20). Such electrical signals from first coil (420) may indicate the real-time position of first coil (420) within the patient (PA).

The electrical signals generated in first coil (420) are communicated along a first electrical conduit (424) and cable (30) to reach the processor of console (12). First electrical conduit (424) extends along the length of catheter (120) and may electrically couple with cable (30) in any suitable fashion. In some versions, first electrical conduit (424) comprises one or more wires. In addition, or in the alternative, first electrical conduit (424) may include one or more traces formed on a substrate of a flex circuit. Alternatively, first electrical conduit (424) may take any other suitable form. In some versions, first electrical conduit (424) extends along the interior of flexible shaft (122). In addition, or in the alternative, a portion or all of first electrical conduit (424) may extend along an exterior of flexible shaft (122).

First coil (420) of this example is oriented such that the windings of coil (420) extend around a first sensor axis (SA₁). First sensor axis (SA₁) is oriented obliquely relative to the longitudinal axis (LA), such that first sensor axis (SA₁) and the longitudinal axis (LA) together define an oblique first angle (Θ₁). By way of example only, first angle (Θ₁) may range from approximately 5 degrees to approximately 45 degrees. Alternatively, any other suitable first angle (Θ₁) may be provided. Because first angle (Θ₁) is oblique in the present example, first coil (420) is configured to generate electrical signals that indicate a "roll" angular position of catheter (120) and end effector (200) about the longitudinal axis (LA). In other words, if first sensor axis (SA₁) were coaxial with the longitudinal axis (LA), first coil (420) the electrical signal generated by first coil (420) may not indicate the "roll" angular position of catheter (120) and end effector (200) about the longitudinal axis (LA).

Second coil (430) of the present example comprises a wire (432) that is wrapped around the exterior of coupling member (410), within exterior recess (412). In some other versions, second coil (430) comprises a flex circuit that includes a combination of a flexible substrate wrapped around the exterior of coupling member (410) and one or more conductive traces on the flexible substrate, where those conductive traces form a coil shape. Second coil (430) is configured to generate electrical signals in response to the presence of alternating magnetic fields that are generated by field generators (20). Such electrical signals from second coil (430) may indicate the real-time position of second coil (430) within the patient (PA).

The electrical signals generated in second coil (430) are communicated along a second electrical conduit (434) and cable (30) to reach the processor of console (12). Second electrical conduit (434) extends along the length of catheter (120) and may electrically couple with cable (30) in any suitable fashion. In some versions, second electrical conduit (434) comprises one or more wires. In addition, or in the alternative, second electrical conduit (434) may include one or more traces formed on a substrate of a flex circuit. Alternatively, second electrical conduit (434) may take any other suitable form. In some versions, second electrical conduit (434) extends along the interior of flexible shaft (122). In addition, or in the alternative, a portion or all of second electrical conduit (434) may extend along an exterior of flexible shaft (122).

Second coil (430) of this example is oriented such that the windings of coil (430) extend around a second sensor axis (SA₂). Second sensor axis (SA₂) is oriented relative to the longitudinal axis (LA) such that second sensor axis (SA₂) and the longitudinal axis (LA) together define a second angle (Θ₂). In some versions, second angle (Θ₂) is the same as first angle (Θ₁). Alternatively, angles (Θ₁, Θ₂) may be different from each other. By way of example only, second angle (Θ₂) may range from approximately 0 degrees to approximately 45 degrees. Alternatively, any other suitable second angle (Θ₂) may be provided. In some versions, due to the orientation of second sensor axis (SA₂), second coil (430) is configured to generate electrical signals that indicate a "roll" angular position of catheter (120) and end effector (200) about the longitudinal axis (LA). In some versions where second sensor axis (SA₂) is not obliquely oriented relative to the longitudinal axis (LA), second sensor axis (SA₂) is obliquely oriented relative to first sensor axis (SA₁).

In the present example, a portion of first coil (420) overlaps with a portion of second coil (430), such that portions of coils (420, 430) are located at the same longitudinal position along the length of coupling member (410). Coils (420, 430) may overlap to any suitable degree. Positioning coils (420, 430) in an overlapping arrangement may reduce the overall length along coupling member (410) collectively occupied by coils (420, 430). In some other versions, coils (420, 430) are longitudinally spaced apart from each other along coupling member (410), such that no portion of first coil (420) overlaps with second coil (430); and vice-versa. In addition, coils (420, 430) are oriented relative to each other such that sensor axes (SA₁, SA₂) are not parallel with each other. Sensor axes (SA1, SA2) cooperate to define a third angle (Θ₃). By way of example only, third angle (Θ₃) may range from approximately 5 degrees to approximately 90 degrees. Alternatively, any other suitable third angle (Θ₃) may be provided. Because coils (420, 430) are oriented differently in relation to each other, the electrical signals from both coils (420, 430) may provide more information on the position and orientation of the coupling member (410) than could otherwise be provided via only one single coil (420, 430). For instance, the electrical signals from both coils (420, 430) may provide information on the position and orientation of coupling member (410) along six degrees of freedom. Information indicating the position and orientation of coupling member (410) may be understood to further indicate the position and orientation of both end effector (200) and the distal end of flexible shaft (122).

Sensor assembly (400) of the present example further includes an outer layer (440) that is positioned about coupling member (410) and coils (420, 430). In some versions, outer layer (440) extends along the full length of coupling member (410). In the present example, outer layer (440) extends along a length that is greater than the length of exterior recess (412) yet less than the full length of coupling member (410). Outer layer (440) is configured and positioned to cover coils (420, 430) and exterior recess (412) without interfering with the functionality of coils (420, 430). Outer layer (440) may prevent edges of exterior recess (412) from snagging or outer layer (440) may serve any other suitable purpose(s). In some versions, outer layer (440) includes a heat-shrink wrap. Alternatively, outer layer (440) may take any other suitable form.

### 3. Example of Three-Axis Position Sensor Assembly

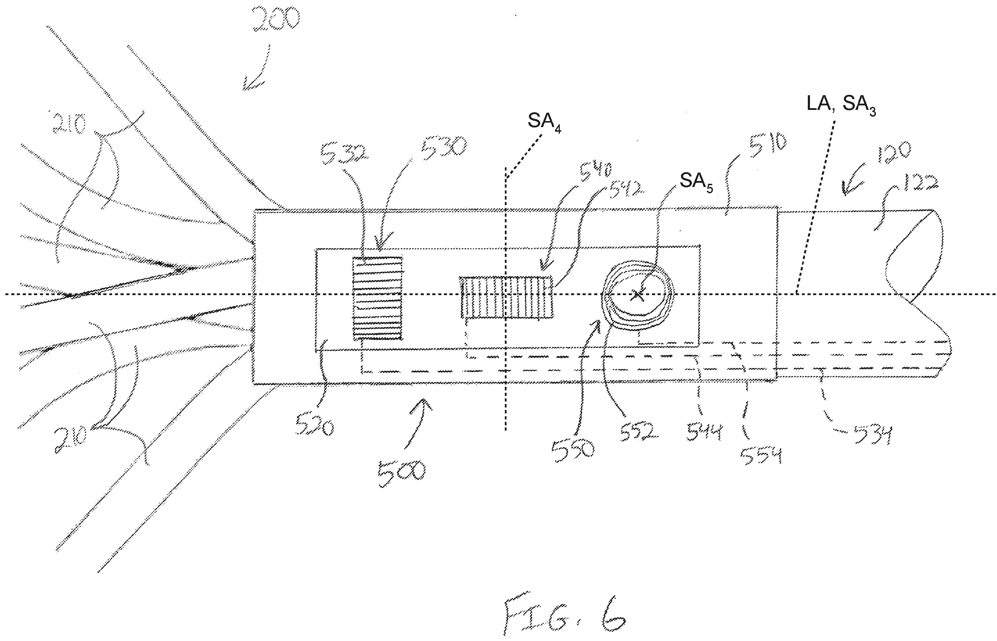

FIG. 6 shows an example of another sensor assembly (500) that may be incorporated into a variation of catheter assembly (100). Sensor assembly (500) of this example includes a coupling member (510), a flex circuit substrate (520), a first coil (530), a second coil (540), and a third coil (550). Coupling member (510) may be constructed and operable like coupling member (230) described above. Coupling member (510) is fixedly secured with the distal end of flexible catheter (122) and the proximal end of end effector (200). In some versions, coupling member (510) is in the form of a rigid plastic (e.g., polyether ether ketone) body. In versions where coupling member (510) is in the form of a rigid body, and catheter shaft (122) includes a steerable section that may be deflected using deflection drive actuator (114), it may be desirable to minimize the length of coupling member (310) in order to bring end effector (200) as close as possible to the point of deflection at this steerable section.

Flex circuit substrate (520) is secured to the exterior of coupling member (510). In some versions, flex circuit substrate (520) wraps about an entire circumference of coupling member (510). In some other versions, flex circuit substrate (520) wraps about only a portion of the circumference of coupling member (510). In the present example, coupling member (510) does not include an exterior recess like coupling members (310, 410). In some other versions, coupling member (510) includes an exterior recess. In some such versions, flex circuit substrate (520) is positioned in such an exterior recess. Flex circuit substrate (520) may be fixedly secured to coupling member (510) via an adhesive, via a shrink wrap or other outer layer, or in any other suitable fashion.

First coil (530) of the present example comprises a conductive trace (532) that is formed on flex circuit substrate (520). Conductive trace (532) is configured such that, when flex circuit substrate (520) is fully secured to coupling member (510), conductive trace (532) forms a coil shape (or a shape that is functionally equivalent to a coil shape). First coil (530) of this example is oriented such that the windings (or functional equivalent of windings) of coil (530) extend around a third sensor axis (SA₃). Third sensor axis (SA₃) is oriented parallel with the longitudinal axis (LA). First coil (530) is configured to generate electrical signals in response to the presence of alternating magnetic fields that are generated by field generators (20). Such electrical signals from first coil (530) may indicate the real-time position of first coil (530) within the patient (PA).

The electrical signals generated in first coil (530) are communicated along a first electrical conduit (534) and cable (30) to reach the processor of console (12). First electrical conduit (534) extends along the length of catheter (120) and may electrically couple with cable (30) in any suitable fashion. In some versions, first electrical conduit (534) comprises one or more wires. In addition, or in the alternative, first electrical conduit (534) may include one or more traces formed on a substrate of a flex circuit. Alternatively, first electrical conduit (534) may take any other suitable form. In some versions, first electrical conduit (534) extends along the interior of flexible shaft (122). In addition, or in the alternative, a portion or all of first electrical conduit (534) may extend along an exterior of flexible shaft (122).

Second coil (540) of the present example comprises a conductive trace (542) that is formed on flex circuit substrate (520). Conductive trace (542) is configured such that, when flex circuit substrate (520) is fully secured to coupling member (510), conductive trace (542) forms a coil shape (or a shape that is functionally equivalent to a coil shape). Second coil (540) of this example is oriented such that the windings (or functional equivalent of windings) of coil (540) extend around a fourth sensor axis (SA₄). Fourth sensor axis (SA₄) is oriented perpendicular to the longitudinal axis (LA), such that fourth sensor axis (SA₄) is also perpendicular to third sensor axis (SA₃). Second coil (540) is configured to generate electrical signals in response to the presence of alternating magnetic fields that are generated by field generators (20). Such electrical signals from second coil (540) may indicate the real-time position of second coil (540) within the patient (PA).

The electrical signals generated in second coil (540) are communicated along a second electrical conduit (544) and cable (30) to reach the processor of console (12). Second electrical conduit (544) extends along the length of catheter (120) and may electrically couple with cable (30) in any suitable fashion. In some versions, second electrical conduit (544) comprises one or more wires. In addition, or in the alternative, second electrical conduit (544) may include one or more traces formed on a substrate of a flex circuit. Alternatively, second electrical conduit (544) may take any other suitable form. In some versions, second electrical conduit (544) extends along the interior of flexible shaft (122). In addition, or in the alternative, a portion or all of second electrical conduit (544) may extend along an exterior of flexible shaft (122).

Third coil (550) of the present example comprises a conductive trace (552) that is formed on flex circuit substrate (520). Conductive trace (552) is configured such that, when flex circuit substrate (520) is fully secured to coupling member (510), conductive trace (552) forms a coil shape (or a shape that is functionally equivalent to a coil shape). Third coil (550) of this example is oriented such that the windings (or functional equivalent of windings) of coil (550) extend around a fifth sensor axis (SA₅). Fifth sensor axis (SA₅) is oriented perpendicular to the longitudinal axis (LA), such that fifth sensor axis (SA₅) is also perpendicular to third sensor axis (SA₃) and perpendicular to fourth sensor axis (SA₄). Third coil (550) is configured to generate electrical signals in response to the presence of alternating magnetic fields that are generated by field generators (20). Such electrical signals from third coil (550) may indicate the real-time position of third coil (550) within the patient (PA).

The electrical signals generated in third coil (550) are communicated along a third electrical conduit (554) and cable (30) to reach the processor of console (12). Third electrical conduit (554) extends along the length of catheter (120) and may electrically couple with cable (30) in any suitable fashion. In some versions, third electrical conduit (554) comprises one or more wires. In addition, or in the alternative, third electrical conduit (554) may include one or more traces formed on a substrate of a flex circuit. Alternatively, third electrical conduit (554) may take any other suitable form. In some versions, third electrical conduit (554) extends along the interior of flexible shaft (122). In addition, or in the alternative, a portion or all of third electrical conduit (554) may extend along an exterior of flexible shaft (122).

Because coils (530, 540, 550) are oriented along respective orthogonal sensor axes (SA₃, SA₄, SA₅), the electrical signals from coils (530, 540, 550) may provide more information on the position and orientation of the coupling member (510) than could otherwise be provided via only one single coil (coils (530, 540, 550). For instance, the electrical signals from all three coils (530, 540, 550) may provide information on the position and orientation of coupling member (510) along six degrees of freedom. Information indicating the position and orientation of coupling member (510) may be understood to further indicate the position and orientation of both end effector (200) and the distal end of flexible shaft (122).

While not shown in FIG. 6, some versions of sensor assembly (500) may further include an outer layer that is positioned about coupling member (510), flex circuit substrate (520), and coils (530, 540, 550). Such an outer layer may extend along all or a portion of the length of coupling member (510). Such an outer layer may cover coils (530, 540, 550) without interfering with the functionality of coils (530, 540, 550). In some versions, such an outer layer includes a heat-shrink wrap. Alternatively, such an outer layer may take any other suitable form. As yet another alternative, such an outer layer may be omitted.

### III. Examples of Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

An apparatus comprising: (a) a catheter shaft assembly having a proximal end and a distal end, the catheter shaft assembly defining a longitudinal axis; (b) an end effector configured to fit in an anatomical passageway within a cardiovascular system of a patient, the end effector including: (i) a proximal end, and (ii) at least one electrode, the at least one electrode being positionable to contact tissue; (c) a coupling member positioned at the distal end of the catheter shaft assembly, the coupling member being further positioned at the proximal end of the end effector, the coupling member including a body having an exterior; and (d) a position sensor assembly, the position sensor assembly including at least one coil, the at least one coil being configured to generate a signal indicative of a position of the at least one coil in response to an alternating magnetic field, at least a portion of the position sensor assembly being positioned about the exterior of the coupling member such that the portion of the position sensor assembly positioned about the exterior of the coupling member is external to the coupling member.

### Example 2

The apparatus of Example 1, the coupling member defining an exterior recess, the portion of the position sensor assembly positioned about the exterior of the coupling member being positioned in the exterior recess.

### Example 3

The apparatus of Example 2, the at least one coil being positioned in the exterior recess.

### Example 4

The apparatus of Example 3, the exterior recess having a depth, the at least one coil having a thickness, the depth of the exterior recess corresponding to the thickness of the at least one coil.

### Example 5

The apparatus of any of Examples 2 through 3, further comprising an outer layer positioned over the position sensor assembly, the outer layer extending along at least a full length of the exterior recess such that the outer layer covers the exterior recess.

### Example 6

The apparatus of Example 5, the outer layer including a shrink wrap.

### Example 7

The apparatus of any of Examples 1 through 6, the at least one coil including a first coil defining a first sensor axis, the first sensor axis being oriented obliquely relative to the longitudinal axis.

### Example 8

The apparatus of Example 7, the at least one coil including a second coil defining a second sensor axis, the second sensor axis being oriented obliquely relative to one or both of the longitudinal axis or the first sensor axis.

### Example 9

The apparatus of Example 8, the second sensor axis and the longitudinal axis cooperating to define an angle ranging from approximately 0 degrees to approximately 45 degrees, the second sensor axis and the first sensor axis cooperating to define an angle ranging from approximately 5 degrees to approximately 90 degrees.

### Example 10

The apparatus of any of Examples 1 through 9, the at least one coil including a first sensor coil and a second sensor coil, at least a portion of the first sensor coil being positioned to overlap at least a portion of the second sensor coil.

### Example 11

The apparatus of any of Examples 1 through 9, the body of the coupling member defining a circumference, the at least one coil being wrapped about the entire circumference of the body.

### Example 12

The apparatus of Example 11, the at least one coil comprising a wire wrapped about the entire circumference of the body.

### Example 13

The apparatus of any of Examples 1 through 12, the position sensor assembly comprising a flex circuit, the flex circuit including a flex circuit substrate and one or more conductive traces.

### Example 14

The apparatus of Example 13, the one or more conductive traces of the flex circuit defining the at least one coil.

### Example 15

The apparatus of any of Examples 1 through 14, the at least one coil including a first coil and a second coil, the first coil defining a first sensor axis, the second coil defining a second sensor axis, the second sensor axis being orthogonal to the first sensor axis.

### Example 16

The apparatus of Example 15, the second sensor axis further being orthogonal to the longitudinal axis.

### Example 17

The apparatus of any of Examples 15 through 16, the first sensor axis being parallel with the longitudinal axis.

### Example 18

The apparatus of any of Examples 15 through 17, the at least one coil further including a third coil, the third coil defining a third sensor axis, the third sensor axis being orthogonal to the first and second sensor axes.

### Example 19

The apparatus of any of Examples 1 through 18, the catheter shaft assembly further comprising one or more electrical conduits, the one or more electrical conduits being electrically coupled with the position sensor assembly.

### Example 20

The apparatus of any of Examples 1 through 19, the coupling member comprising a rigid material.

### Example 21

The apparatus of any of Examples 1 through 20, the catheter shaft assembly having a steerable portion, the steerable portion being operable to deflect the end effector laterally away from the longitudinal axis.

### Example 22

The apparatus of any of Examples 1 through 21, the at least one electrode being operable to provide electrophysiology (EP) mapping of tissue.

### Example 23

The apparatus of any of Examples 1 through 22, the at least one electrode being operable to ablate tissue.

### Example 24

The apparatus of any of Examples 1 through 23, the end effector being configured to transition between an expanded state and a non-expanded state.

### Example 25

The apparatus of Example 24, the end effector further comprising one or more resilient members configured to resiliently urge the end effector toward the expanded state.

### Example 26

An apparatus comprising: (a) a catheter shaft assembly having a proximal end and a distal end, the catheter shaft assembly defining a longitudinal axis; (b) an end effector configured to fit in an anatomical passageway within a cardiovascular system of a patient, the end effector including: (i) a proximal end, and (ii) at least one electrode, the at least one electrode being positionable to contact tissue; (c) a coupling member positioned at the distal end of the catheter shaft assembly, the coupling member being further positioned at the proximal end of the end effector, the coupling member including a body having an exterior region including an exterior recess; and (d) a position sensor assembly, the position sensor assembly including a coil positioned about the exterior region of the coupling member within the exterior recess such that the coil is external to the coupling member, the coil being configured to generate a signal indicative of a position of the coil in response to an alternating magnetic field.

### Example 27

An apparatus comprising: (a) a catheter shaft assembly having a proximal end and a distal end, the catheter shaft assembly defining a longitudinal axis; (b) an end effector configured to fit in an anatomical passageway within a cardiovascular system of a patient, the end effector including: (i) a proximal end, and (ii) at least one electrode, the at least one electrode being positionable to contact tissue; (c) a coupling member positioned at the distal end of the catheter shaft assembly, the coupling member being further positioned at the proximal end of the end effector, the coupling member including a body having an exterior region; and (d) a position sensor assembly, the position sensor assembly including: (i) a first coil positioned about the exterior of the coupling member such that the first coil is external to the coupling member, the first coil being configured to generate a signal indicative of a position of the first coil in response to an alternating magnetic field, the first coil defining a first sensor axis, the first sensor axis being oriented obliquely relative to the longitudinal axis, and (ii) a second coil positioned about the exterior of the coupling member such that the second coil is external to the coupling member, the second coil being configured to generate a signal indicative of a position of the second coil in response to an alternating magnetic field, the second sensor axis being oriented obliquely relative to one or both of the longitudinal axis or the first sensor axis.

### Example 28

An apparatus comprising: (a) a catheter shaft assembly having a proximal end and a distal end, the catheter shaft assembly defining a longitudinal axis; (b) an end effector configured to fit in an anatomical passageway within a cardiovascular system of a patient, the end effector including: (i) a proximal end, and (ii) at least one electrode, the at least one electrode being positionable to contact tissue; (c) a coupling member positioned at the distal end of the catheter shaft assembly, the coupling member being further positioned at the proximal end of the end effector, the coupling member including a body having an exterior region; and (d) a position sensor assembly, the position sensor assembly including: (i) a first coil positioned about the exterior of the coupling member such that the first coil is external to the coupling member, the first coil being configured to generate a signal indicative of a position of the first coil in response to an alternating magnetic field, the first coil defining a first sensor axis, the first sensor axis being oriented parallel to the longitudinal axis, (ii) a second coil positioned about the exterior of the coupling member such that the second coil is external to the coupling member, the second coil being configured to generate a signal indicative of a position of the second coil in response to an alternating magnetic field, the second coil defining a second sensor axis, the second sensor axis being orthogonal to the longitudinal axis, and (iii) a third coil positioned about the exterior of the coupling member such that the third coil is external to the coupling member, the third coil being configured to generate a signal indicative of a position of the third coil in response to an alternating magnetic field, the third coil defining a third sensor axis, the third sensor axis being orthogonal to the longitudinal axis, the third sensor axis being further orthogonal to the second sensor axis.

### IV. Miscellaneous

Any of the instruments described herein may be cleaned and sterilized before or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, hydrogen peroxide, peracetic acid, and vapor phase sterilization, either with or without a gas plasma, or steam.

It should be understood that any of the examples described herein may include various other features in addition to or in lieu of those described above. By way of example only, any of the examples described herein may also include one or more of the various features disclosed in any of the various references cited.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

Having shown and described various versions of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, versions, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. An apparatus (100) comprising:
(a) a catheter shaft assembly (122) including a proximal end and a distal end, the catheter shaft assembly defining a longitudinal axis (LA);
(b) an end effector (200) configured to fit in an anatomical passageway within a cardiovascular system of a patient, the end effector including:
(i) a proximal end, and
(ii) at least one electrode, the at least one electrode being positionable to contact tissue;
(c) a coupling member (230) positioned at the distal end of the catheter shaft assembly, the coupling member being further positioned at the proximal end of the end effector, the coupling member including a body including an exterior, the body of the coupling member defining a circumference; and
(d) a position sensor assembly (300), the position sensor assembly including at least one coil (320), the at least one coil being configured to generate a signal indicative of a position of the at least one coil in response to an alternating magnetic field, at least a portion of the position sensor assembly being positioned about the exterior of the coupling member such that the portion of the position sensor assembly positioned about the exterior of the coupling member is external to the coupling member, **characterized by** the at least one coil being wrapped about the entire circumference of the body of the coupling member.

2. The apparatus of claim 1, the coupling member defining an exterior recess (312), the portion of the position sensor assembly positioned about the exterior of the coupling member being positioned in the exterior recess.

3. The apparatus of claim 2, the at least one coil being positioned in the exterior recess, optionally the exterior recess including a depth, the at least one coil including a thickness, the depth of the exterior recess corresponding to the thickness of the at least one coil.

4. The apparatus of claim 2, further comprising an outer layer (330) positioned over the position sensor assembly, the outer layer extending along at least a full length of the exterior recess such that the outer layer covers the exterior recess, optionally the outer layer including a shrink wrap.

5. The apparatus of any preceding claim, the at least one coil including a first coil (420) defining a first sensor axis (SA₁), the first sensor axis being oriented obliquely relative to the longitudinal axis, optionally the at least one coil including a second coil (430) defining a second sensor axis (SA₂), the second sensor axis being oriented obliquely relative to one or both of the longitudinal axis or the first sensor axis, further optionally the second sensor axis and the longitudinal axis cooperating to define an angle ranging from 0 degrees to 45 degrees, the second sensor axis and the first sensor axis cooperating to define an angle ranging from 5 degrees to 90 degrees.

6. The apparatus of any one of claims 1 to 4, the at least one coil including a first sensor coil (420) and a second sensor coil (430), at least a portion of the first sensor coil being positioned to overlap at least a portion of the second sensor coil.

7. The apparatus of any of claims 1 to 5, the at least one coil comprising a wire (322) wrapped about the entire circumference of the body of the coupling member.

8. The apparatus of any preceding claim, the position sensor assembly comprising a flex circuit, the flex circuit including a flex circuit substrate (520) and one or more conductive traces (532, 542, 522), optionally the one or more conductive traces of the flex circuit defining the at least one coil.

9. The apparatus of any one of claims 1 to 4, the at least one coil including a first coil (420) and a second coil (430), the first coil defining a first sensor axis (SA₁), the second coil defining a second sensor axis (SA₂), the second sensor axis being orthogonal to the first sensor axis.

10. The apparatus of claim 9, the second sensor axis further being orthogonal to the longitudinal axis, and/or the first sensor axis being parallel with the longitudinal axis.

11. The apparatus of claim 9, the at least one coil further including a third coil (550), the third coil defining a third sensor axis (SA₃), the third sensor axis being orthogonal to the first and second sensor axes.

12. The apparatus of any preceding claim, the catheter shaft assembly further comprising one or more electrical conduits (324), the one or more electrical conduits being electrically coupled with the position sensor assembly.

13. The apparatus of any preceding claim, the coupling member comprising a rigid material.

14. The apparatus of any preceding claim, the catheter shaft assembly including a steerable portion, the steerable portion being operable to deflect the end effector laterally away from the longitudinal axis.

15. The apparatus of any preceding claim, the at least one electrode being operable to (i) provide electrophysiology (EP) mapping of tissue or (ii) ablate tissue.

16. The apparatus of any preceding claim, the end effector being configured to transition between an expanded state and a non-expanded state, optionally the end effector further comprising one or more resilient members configured to resiliently urge the end effector toward the expanded state.

## Patentansprüche

1. Einrichtung (100), umfassend:
(a) eine Katheterschaftanordnung (122), einschließlich eines proximalen Endes und eines distalen Endes, wobei die Katheterschaftanordnung eine Längsachse (LA) definiert;
(b) einen Endeffektor (200), der konfiguriert ist, um in einen anatomischen Durchgang innerhalb eines kardiovaskulären Systems eines Patienten zu passen, wobei der Endeffektor einschließt:
(i) ein proximales Ende und
(ii) mindestens eine Elektrode, wobei die mindestens eine Elektrode positionierbar ist, um Gewebe zu berühren;
(c) ein Kopplungselement (230), das an dem distalen Ende der Katheterschaftanordnung positioniert ist, wobei das Kopplungselement ferner an dem proximalen Ende des Endeffektors positioniert ist, wobei das Kopplungselement einen Körper einschließt, der eine Außenseite einschließt, wobei der Körper des Kopplungselements einen Umfang definiert; und
(d) eine Positionssensoranordnung (300), wobei die Positionssensoranordnung mindestens eine Spule (320) einschließt, wobei die mindestens eine Spule konfiguriert ist, um als Reaktion auf ein magnetisches Wechselfeld ein Signal zu erzeugen, das eine Position der mindestens einen Spule anzeigt, wobei mindestens ein Abschnitt der Positionssensoranordnung derart um die Außenseite des Kopplungselements herum positioniert ist, dass der Abschnitt der Positionssensoranordnung, der um die Außenseite des Kopplungselements herum positioniert ist, sich außerhalb des Kopplungselements befindet, **gekennzeichnet durch** die mindestens eine Spule, die um den gesamten Umfang des Körpers des Kopplungselements herum gewickelt ist.

2. Einrichtung nach Anspruch 1, wobei das Kopplungselement eine äußere Aussparung (312) definiert, wobei der Abschnitt der Positionssensoranordnung, der um die Außenseite des Kopplungselements herum angeordnet ist, in der äußeren Aussparung angeordnet ist.

3. Einrichtung nach Anspruch 2, wobei die mindestens eine Spule in der äußeren Aussparung positioniert ist, wobei optional die äußere Aussparung eine Tiefe einschließt, wobei die mindestens eine Spule eine Dicke einschließt, wobei die Tiefe der äußeren Aussparung der Dicke der mindestens einen Spule entspricht.

4. Einrichtung nach Anspruch 2, ferner umfassend eine Außenschicht (330), die über der Positionssensoranordnung positioniert ist, wobei sich die Außenschicht entlang mindestens einer vollen Länge der äußeren Aussparung derart erstreckt, dass die Außenschicht die äußere Aussparung bedeckt, wobei optional die Außenschicht eine Schrumpffolie einschließt.

5. Einrichtung nach einem der vorstehenden Ansprüche, wobei die mindestens eine Spule eine erste Spule (420) einschließt, die eine erste Sensorachse (SA₁) definiert, wobei die erste Sensorachse schräg relativ zu der Längsachse ausgerichtet ist, wobei optional die mindestens eine Spule eine zweite Spule (430) einschließt, die eine zweite Sensorachse (SA₂) definiert, wobei die zweite Sensorachse schräg relativ zu einer oder beiden der Längsachse oder der ersten Sensorachse ausgerichtet ist, wobei ferner optional die zweite Sensorachse und die Längsachse zusammenwirken, um einen Winkel in einem Bereich von 0 Grad bis 45 Grad zu definieren, wobei die zweite Sensorachse und die erste Sensorachse zusammenwirken, um einen Winkel in einem Bereich von 5 Grad bis 90 Grad zu definieren.

6. Einrichtung nach einem der Ansprüche 1 bis 4, wobei die mindestens eine Spule eine erste Sensorspule (420) und eine zweite Sensorspule (430) einschließt, wobei mindestens ein Abschnitt der ersten Sensorspule positioniert ist, um mindestens einen Abschnitt der zweiten Sensorspule zu überlappen.

7. Einrichtung nach einem der Ansprüche 1 bis 5, die mindestens eine Spule umfassend einen Draht (322), der um den gesamten Umfang des Körpers des Kopplungselements herum gewickelt ist.

8. Einrichtung nach einem der vorstehenden Ansprüche, die Positionssensoranordnung umfassend eine Flexschaltung, wobei die Flexschaltung ein Flexschaltungssubstrat (520) und eine oder mehrere leitfähige Leiterbahnen (532, 542, 522) einschließt, wobei optional die eine oder die mehreren leitfähigen Leiterbahnen der Flexschaltung die mindestens eine Spule definieren.

9. Einrichtung nach einem der Ansprüche 1 bis 4, wobei die mindestens eine Spule eine erste Spule (420) und eine zweite Spule (430) einschließt, wobei die erste Spule eine erste Sensorachse (SA₁) definiert, wobei die zweite Spule eine zweite Sensorachse (SA₂) definiert, wobei die zweite Sensorachse orthogonal zu der ersten Sensorachse ist.

10. Einrichtung nach Anspruch 9, wobei die zweite Sensorachse ferner orthogonal zu der Längsachse ist und/oder die erste Sensorachse parallel zu der Längsachse ist.

11. Einrichtung nach Anspruch 9, wobei die mindestens eine Spule ferner eine dritte Spule (550) einschließt, wobei die dritte Spule eine dritte Sensorachse (SA₃) definiert, wobei die dritte Sensorachse orthogonal zu der ersten und der zweiten Sensorachse ist.

12. Einrichtung nach einem der vorstehenden Ansprüche, die Katheterschaftanordnung ferner umfassend eine oder mehrere elektrische Leitungen (324), wobei die eine oder die mehreren elektrischen Leitungen mit der Positionssensoranordnung elektrisch gekoppelt sind.

13. Einrichtung nach einem der vorstehenden Ansprüche, das Kopplungselement umfassend ein starres Material.

14. Einrichtung nach einem der vorstehenden Ansprüche, wobei die Katheterschaftanordnung einen lenkbaren Abschnitt einschließt, wobei der lenkbare Abschnitt betätigbar ist, um den Endeffektor lateral von der Längsachse weg abzulenken.

15. Einrichtung nach einem der vorstehenden Ansprüche, wobei die mindestens eine Elektrode betätigbar ist, um (i) ein elektrophysiologisches (EP) Mapping von Gewebe bereitzustellen oder (ii) Gewebe zu ablatieren.

16. Einrichtung nach einem der vorstehenden Ansprüche, wobei der Endeffektor konfiguriert ist, um zwischen einem expandierten Zustand und einem nicht expandierten Zustand zu wechseln, optional der Endeffektor ferner umfassend ein oder mehrere elastische Elemente, die konfiguriert sind, um den Endeffektor elastisch zu dem expandierten Zustand hin zu drängen.

## Revendications

1. Appareil (100) comprenant :
(a) un ensemble tige de cathéter (122) comportant une extrémité proximale et une extrémité distale, l'ensemble tige de cathéter définissant un axe longitudinal (LA) ;
(b) un effecteur terminal (200) conçu pour s'ajuster dans une voie de passage anatomique au sein d'un système cardiovasculaire d'un patient, l'effecteur terminal comportant :
(i) une extrémité proximale, et
(ii) au moins une électrode, l'au moins une électrode étant positionnable pour venir en contact avec un tissu ;
(c) un élément d'accouplement (230) positionné au niveau de l'extrémité distale de l'ensemble tige de cathéter, l'élément d'accouplement étant en outre positionné au niveau de l'extrémité proximale de l'effecteur terminal, l'élément d'accouplement comportant un corps comportant un extérieur, le corps de l'élément d'accouplement définissant une circonférence ; et
(d) un ensemble capteur de position (300), l'ensemble capteur de position comportant au moins une bobine (320), l'au moins une bobine étant configurée pour générer un signal indiquant une position de l'au moins une bobine en réponse à un champ magnétique alternatif, au moins une partie de l'ensemble capteur de position étant positionnée autour de l'extérieur de l'élément d'accouplement de telle sorte que la partie de l'ensemble capteur de position positionnée autour de l'extérieur de l'élément d'accouplement est externe à l'élément d'accouplement, **caractérisé par** l'au moins une bobine étant enveloppée autour de la circonférence entière du corps de l'élément d'accouplement.

2. Appareil selon la revendication 1, l'élément d'accouplement définissant un évidement extérieur (312), la partie de l'ensemble capteur de position positionnée autour de l'extérieur de l'élément d'accouplement étant positionnée dans l'évidement extérieur.

3. Appareil selon la revendication 2, l'au moins une bobine étant positionnée dans l'évidement extérieur, facultativement l'évidement extérieur comportant une profondeur, l'au moins une bobine comportant une épaisseur, la profondeur de l'évidement extérieur correspondant à l'épaisseur de l'au moins une bobine.

4. Appareil selon la revendication 2, comprenant en outre une couche externe (330) positionnée par-dessus l'ensemble capteur de position, la couche externe s'étendant le long d'au moins une longueur complète de l'évidement extérieur de telle sorte que la couche externe couvre l'évidement extérieur, facultativement la couche externe comportant une enveloppe rétractable.

5. Appareil selon l'une quelconque revendication précédente, l'au moins une bobine comportant une première bobine (420) définissant un premier axe de capteur (SA₁), le premier axe de capteur étant orienté en oblique par rapport à l'axe longitudinal, facultativement l'au moins une bobine comportant une deuxième bobine (430) définissant un deuxième axe de capteur (SA₂), le deuxième axe de capteur étant orienté en oblique par rapport à l'un et/ou l'autre de l'axe longitudinal ou du premier axe de capteur, facultativement en outre le deuxième axe de capteur et l'axe longitudinal coopérant pour définir un angle allant de 0 degré à 45 degrés, le deuxième axe de capteur et le premier axe de capteur coopérant pour définir un angle allant de 5 degrés à 90 degrés.

6. Appareil selon l'une quelconque des revendications 1 à 4, l'au moins une bobine comportant une première bobine de capteur (420) et une seconde bobine de capteur (430), au moins une partie de la première bobine de capteur étant positionnée pour chevaucher au moins une partie de la seconde bobine de capteur.

7. Appareil selon l'une quelconque des revendications 1 à 5, l'au moins une bobine comprenant un fil (322) enveloppé autour de la circonférence entière du corps de l'élément d'accouplement.

8. Appareil selon l'une quelconque revendication précédente, l'ensemble capteur de position comprenant un circuit souple, le circuit souple comportant un substrat de circuit souple (520) et un ou plusieurs tracés conducteurs (532, 542, 522), facultativement le ou les tracés conducteurs du circuit souple définissant l'au moins une bobine.

9. Appareil selon l'une quelconque des revendications 1 à 4, l'au moins une bobine comportant une première bobine (420) et une deuxième bobine (430), la première bobine définissant un premier axe de capteur (SA₁), la deuxième bobine définissant un deuxième axe de capteur (SA₂), le deuxième axe de capteur étant orthogonal au premier axe de capteur.

10. Appareil selon la revendication 9, le deuxième axe de capteur étant en outre orthogonal à l'axe longitudinal, et/ou le premier axe de capteur étant parallèle à l'axe longitudinal.

11. Appareil selon la revendication 9, l'au moins une bobine comportant en outre une troisième bobine (550), la troisième bobine définissant un troisième axe de capteur (SA₃), le troisième axe de capteur étant orthogonal aux premier et deuxième axes de capteur.

12. Appareil selon l'une quelconque revendication précédente, l'ensemble tige de cathéter comprenant en outre un ou plusieurs conduits électriques (324), le ou les conduits électriques étant couplés électriquement à l'ensemble capteur de position.

13. Appareil selon l'une quelconque revendication précédente, l'élément d'accouplement comprenant un matériau rigide.

14. Appareil selon l'une quelconque revendication précédente, l'ensemble tige de cathéter comportant une partie orientable, la partie orientable étant fonctionnelle pour dévier l'effecteur terminal latéralement à l'écart de l'axe longitudinal.

15. Appareil selon l'une quelconque revendication précédente, l'au moins une électrode étant fonctionnelle pour (i) fournir une cartographie d'électrophysiologie (EP) d'un tissu ou (ii) ablater un tissu.

16. Appareil selon l'une quelconque revendication précédente, l'effecteur terminal étant conçu pour effectuer une transition entre un état expansé et un état non expansé, facultativement l'effecteur terminal comprenant en outre un ou plusieurs éléments élastiques conçus pour presser élastiquement l'effecteur terminal en direction de l'état expansé.
